## Europäisches Patentamt
## European Patent Office
## Office européen des brevets

(19)

(11) Veröffentlichungsnummer: **0 127 025**
**B1**

(12) **EUROPÄISCHE PATENTSCHRIFT**

(45) Veröffentlichungstag der Patentschrift:
19.07.89

(51) Int. Cl.⁴ : **C 07 K 3/12, A 61 K 35/16,
A 61 K 37/02**

(21) Anmeldenummer: 84105221.0

(22) Anmeldetag: 09.05.84

(54) Verfahren zur Herstellung eines Antihämophiliefaktor-Konzentrats.

(30) Priorität: 20.05.83 DE 3318521

(43) Veröffentlichungstag der Anmeldung:
05.12.84 Patentblatt 84/49

(45) Bekanntmachung des Hinweises auf die Patenterteilung: 19.07.89 Patentblatt 89/29

(84) Benannte Vertragsstaaten:
AT BE CH DE FR GB IT LI LU NL SE

(56) Entgegenhaltungen:
AT-A- 349 639
US-A- 4 188 318
ARZNEIMITTELFORSCHUNG / DRUG RES., Band 31(I), Nr. 4, April 1981, Seiten 619-622, Aulendorf, DE; N. HEIMBURGER et al.: "Faktor VIII-Konzentrat, hochgereinigt und in Lösung erhitzt"

(73) Patentinhaber: SCHWAB & CO. GES.M.B.H.
Doerenkampgasse 4
A-1100 Wien (AT)
BE CH FR GB IT LI LU NL SE AT
Lentia Gesellschaft mit beschränkter Haftung
Arabellastrasse 4 Postfach 81 05 08
D-8000 München 81 (DE)
DE

(72) Erfinder: Frolik, Manfred
Kierlingergasse 19
A-3420 Kritzendorf (AT)
Erfinder: Kaltschmid, Helmut, Dr.
Dammgasse 11
A-2540 Bad Vöslau (AT)

(74) Vertreter: Kunz, Ekkehard, Dr.
Chemie Holding AG Patentwesen St. Peter-Strasse 25
A-4021 Linz (AT)

## Beschreibung

Die Erfindung betrifft ein Verfahren zur Herstellung eines gereinigten Antihämophiliefaktor (AHF, Faktor VIII)-Konzentrats mit einer Konzentration von mindestens 25 E AHF/ml und einer spezifischen Aktivität .von mindestens 1,5 E AHF/mg Protein aus menschlichem Blutplasma durch eine Kombination von Maßnahmen wie durch Kryopräzipitieren, Fällen, Reinigen mit Proteinfällungsmitteln, Zentrifugieren und Lyophilisieren.

Zu den wichtigsten physiologischen Eigenschaften des Blutes zählt dessen Gerinnungsfähigkeit, die gewährleistet, daß Blut bei Verletzungen mit offenen Wunden oder bei internen Blutungen gestillt wird. Am Gerinnungsvorgang, der enzymatisch kontrolliert wird und der über mehrere Reaktionsschritte abläuft, können bis zu zwölf plasmatische Faktoren und zusätzliche Thrombozyten-oder Zellelemente beteiligt sein.

Unter den Gerinnungsfaktoren nimmt der AHF einen besonderen Rang ein. Im Humanplasma ist der AHF in der Regel in einer sehr geringen Konzentration von etwa 10 mg/l Plasma vorhanden. Fehlen und Mangel dieses Faktors sind vererblich und bilden die Ursache der häufigsten angeborenen Bluterkrankung, der Hämophilie A, die nur durch Substitution des fehlenden Plasmafaktors VIII behandelt werden kann. Die Isolierung und Reinigung des AHF ist daher für die Bereitstellung von Präparaten zur Behandlung von Hämophilie-Patienten von allergrößter Bedeutung.

Der AHF ist ein hochmolekulares Protein, dessen Reindarstellung wegen der ausgeprägten chemischen Labilität bisher nicht gelungen ist. Es sind aber bereits verschiedene Verfahren für die Herstellung von therapeutisch wirksamen AHF-Konzentraten beschrieben worden. Bekannte Präparate mit niedriger Konzentration an Faktor VIII bezogen auf das Gesamtprotein sind insbesondere die Kryopräzipitate, die mittels eines Ausfrierverfahrens gewonnen werden, welches auf der abgestuften Löslichkeit der Plasmaproteine nach dem Einfrieren und Wiederauftauen beruht. Bei diesem Verfahren wird tiefgefrorenes Plasma in einem niedrigen Temperaturbereich von etwa 0 °C bis 10 °C langsam aufgetaut, das entstandene Kryopräzipitat durch Filtrieren oder Zentrifugieren abgetrennt und ohne weitere Reinigungsoperationen gegebenenfalls durch Wiederauflösen und Gefriertrocknen in eine haltbare Form gebracht (vgl. dazu J. Pool et al., Nature 203, 1964, S 312 ff.).

Diese Kryopräzipitate enthalten neben dem AHF noch andere Plasmaproteine in größeren Mengen, beispielsweise Fibrinogen, Plasminogen und Plasmin. Insbesondere der hohe Fibrinogengehalt bewirkt bei diesen Präparaten eine geringe Löslichkeit, die zur Folge hat, daß für die Verabreichung einer gewöhnlichen Einzeldosis an AHF von beispielsweise 500 E (Einheiten) bis zu 100 ml Flüssigkeit erforderlich sind. Große Flüssigkeitsmengen stellen einerseits eine Belastung für Herz und Kreislauf dar und machen andererseits die Hämophilie-Patienten, die auf die ständige Substitution des fehlenden Blutfaktors angewiesen sind, von Infusionen und ärztlicher Hilfe abhängig. Es ist daher vorteilhaft Konzentrate zu verabreichen, deren Volumen 20 bis 30 ml Flüssigkeit nicht übersteigt und die sich der Patient ohne ärztliche Hilfe selbst injizieren kann. Zu diesem Zweck war es notwendig, AHF-Injektionslösungen mit höherem Reinheitsgrad und besserer Löslichkeit zu finden, die in einem Volumen von etwa 20 bis 30 ml Flüssigkeit etwa 500 E AHF enthalten.

In der AT-A-349.639 wird ein Verfahren zur Herstellung eines gereinigten AHF-Präparates beschrieben, wobei die Reinigung darauf beruht, daß Rohkryopräzipitat mit destilliertem Wasser extrahiert wird und die erhaltene Lösung einer Kältefällung bei 1-2 °C unterworfen wird. Das erhaltene AHF-Präparat enthält jedoch noch immer derart viele Verunreinigungen, daß es nach der Gefriertrocknung nur zu Lösungen mit 10 E/ml gelöst werden kann.

In der DE-OS-2,516.186 ist ein Reinigungs- und Anreicherungsverfahren beschrieben, dessen wesentliche Merkmale darin bestehen, daß das gesammelte Rohkryopräzipitat mit einem Citratpuffer extrahiert wird und aus diesem Pufferextrakt ein Großteil des Fibrinogen und anderer Begleitproteine durch Einstellen eines pH-Wertes von 6.0 bis 6.8 und einer Temperatur von 0 °C bis 20 °C ohne Zugabe eines Fällungsmittels ausgefällt wird. Aus dem neutralisierten Überstand kann dann durch Sterilfiltrieren und Lyophilisieren ein AHF-Präparat gewonnen werden, welches zwar wesentlich besser löslich ist als das unbehandelte Kryopräzipitat, aber noch Verunreinigungen und Begleitproteine, beispielsweise im Verlaufe der Fällung denaturierten AHF, enthält und eine spezifische Aktivität von weniger als 1 E pro mg Protein aufweist.

In einer Weiterentwicklung ist in der US-PS-4,294.826 ein zweistufiges Reinigungs-und Anreicherungsverfahren aus dem Kryopräzipitat beschrieben, bei dem in der ersten Stufe eine Pufferlösung des Kryoprazipitats einer Fibrinogenfällung unter den in der DE-OS-2,516.186 angegebenen Bedingungen unterzogen wird, worauf die so vorgereinigte und an AHF angereicherte Lösung in einer zweiten Stufe durch eine neuerliche pH-Fällung bei pH 5.0 bis 6.0 bei einer Temperatur von 0° bis 14 °C und Abtrennen der unter diesen Bedingungen ausgefällten Begleitproteine weitergereinigt wird. Nach der Rückstellung des pH-Wertes im Überstand in den Neutralitätsbereich wird entweder die Lösung selbst oder ein Lyophilisat davon zum fertigen AHF-Präparat verarbeitet. Mit diesem Verfahren wird zwar der Reinigungseffekt verbessert und ein Produkt erhalten, welches eine spezifische Aktivität von mindestens .1 E pro mg Protein aufweist, aber neben dem Umstand, daß in der US-PS-4,294.826 keine Angaben über die Ausbeute enthalten sind, hat das Verfshren den Nachteil, daß sämtliche Lösungsmittel, welche im Verlaufe des Verfahrens von der Auflösung des Kryopräzipitats bis zur abschließenden Neutralisation zugegeben werden, zur Herstellung eines Lyophilisats oder einer konzentrierten AHF-Lösung ganz oder teilweise durch Gefriertrocknung wieder entfernt werden wüssen. Die Entfernung von größeren Lösungsmittelvolumina durch Lyophilisation ist aber zeitraubend und kostspielig. Desgleichen bleiben sämtliche Verunreinigungen organischer und anorganischer Natur, die nicht bei der zweifachen pH-Fällung ausfallen und abgetrennt werden, beispielsweise sämtliche Puffer- und Neutralisationssalze, in der fertigen AHF-Lösung oder im Lyophilisat zurück.

Andererseits ist in der Arbeit von N. Heimburger et al. in der Zeitschrift Arzneimittelforschung 31 (1) Nr. 4 (1981), S.619-623 ein Verfahren zur Herstellung eines hochgereinigten AHF-Präparats beschrieben, bei dem das Fibrinogen aus einer Lösung des Kryopräzipitats durch fraktionierte Fällung mit Glycin entfernt und der AHF über eine nachfolgende Natriumchlorid-Fällung aus dem Glycinüberstand ausgefällt wird. Bei diesem Verfahren erhält man zwar hochgereinigte Präparate, muß jedoch den Nachteil von Ausbeuteverlusten in Kauf nehmen, da bei der fraktionierten Glycinfällung der AHF teilweise mitgefällt wird.

Der Erfindung liegt nun die Aufgabe zugrunde, ein Verfahren zu schaffen, welches unter Vermeidung der den bekannten Verfahren anhaftenden Nachteile eine wirtschaftlich Herstellung eines weitgehend reinen AHF-Präparats mit einer hohen Faktor VIII-Konzentration in guter Ausbeute aus Humanplasma ermöglicht.

Diese Aufgabe wird beim erfindungsgemäßen Verfahren dadurch gelöst, daß der größte Teil des Fibrinogen und anderer Begleitproteine durch eine pH-Fällung entfernt und in der letzten Reinigungsstufe der AHF durch selektive Reinigungsfällung mittels Proteinfällungsmittel aus der vorgereinigten Lösung isoliert wird.

Gegenstand der vorliegenden Erfindung ist demnach ein Verfahren zur Herstellung eines AHF-Konzentrats mit einer Konzentration von mindestens 25 E pro ml und einer spezifischen Aktivität von mindestens 1.5 AHF pro mg Protein aus menschlichem Blutplasma durch eine Kombination von Maßnahmen wie durch Kryopräzipitieren, Fällen, Reinigen mit Proteinfällungsmitteln, Zentrifugieren und Lyophilisieren, welches dadurch gekennzeichnet ist, daß man

a) das Kryopräzipitat in einem Puffer geringer lonenstärke auflöst, in der Lösung einen pH-Wert von 6.0 bis 6.8 sowie eine Temperatur von 5° bis 15 °C einstellt und die dabei entstandene Fällung vom Überstand abtrennt, sodann

b) den so vorgereinigten und an AHF in Bezug auf die Begleitproteine angereicherten Überstand neutralisiert, durch aufeinanderfolgende Zugabe von Natriumchlorid bis zu einer Konzentration von etwa 15 Gew. % und von Glycin bis zu einer Menge von etwa 10 Gew.% einer Reinigungsfällung unterzieht, worauf der das AHF-Konzentrat enthaltende Niederschlag abgetrennt, gegebenenfalls unter Zusatz von Stabilisatoren in einem physiologischen Puffer wiedergelöst und durch Tieffrieren und Lyophilisieren in haltbare Form gebracht wird.

Zur Auflösung des Kryopräzipitats eignen sich Pufferlösungen mit geringer lonenstärke und Konzentration, beispielsweise Citratpuffer mit einer Konzentration von 0.05 bis 0.5 Gew.% Tri-Natriumcitrat, vorzugsweise kann eine 0.1 Gew.%ige Tri-Natriumcitratpufferlösung verwendet werden. Das Auflösen kann bei Raumtemperatur geschehen, jedoch kann bei großeren Kryopräzipitatmengen gelindes Erwärmen auf 30 bis 40°C zur Beschleunigung des Lösungsvorgangs zweckmäßig sein.

Die Lösung des Kryopräzipitats wird zur Ausfällung des größten Teils an Fibrinogen durch Zugabe von verdünnten anorganischen oder organischen Säuren, beispielsweise verdünnter Salzsäure, Essigsäure oder Citronensäure auf einen pH-Wert von etwa 6.0 bis 6.8 eingestellt und rasch auf Temperaturen von 5° bis 15 °C abgekühlt.

In einer bevorzugten Ausführungsform kann die Fibrinogenfällung durch Ansäuern der Kryopräzipitatlösung mit etwa 0.1 n Salzsäure oder etwa 1 Gew.%iger Citronensäure unter Rühren auf einen pH-Wert von 6.2 bis 6.4, wobei wiederum ein pH-Wert von 6.30 besonders bevorzugt ist, bei einer Temperatur von 10° bis 15 °C durchgeführt werden.

Nach längerem Stehen, fällt ein faseriger Niederschlag aus, der auf übliche Weise durch Filtrieren, Dekantieren oder bevorzugt durch Zentrifugieren abgetrennt werden kann.

Unter den angegebenen Bedingungen fallen Begleitproteine des AHF, vor allem der größte Teil des Fibrinogen, aber auch CIP (Cold-insoluble proteins), Plasminogen und Plasmin aus, während der AHF nicht mitgefällt wird und im klaren Überstand bzw. Filtrat zurückbleibt.

Der pH-Wert des Überstandes bzw. des Filtrats wird sodann vorzugsweise mit einer verdünnten Base, zweckmäßig mit etwa 1 n Natronisuge auf einen pH-Wert von 6.8 bis 7.5, vorzugsweise auf einen pH-Wert von 7.0 eingestellt.

Die Reinigungsfällung des AHF wird erfindungsgemäß durch abgestufte Zugabe von Proteinfällungsmitteln durchgeführt, indem der neutralisierte Überstand bzw. das Filtrat der Fibrinogenfällung mit Natriumchlorid unter Rühren bis zu einer Konzentration von etwa 15 Gew.% versetzt wird und anschließend durch langsame Zugabe von Glycin bis zu einer Konzentration von etwa 10 Gew.%, die AHF-Fällung vervollständigt wird. Zweckmäßig wird während der Glycinfällung durch Zugabe verdünnter Basen, beispielsweise von 0.1 n NaOH, der pH-Wert im Neutralitätsbereich, vorzugsweise im Bereich von 6.8 bis 7.5, ganz bevorzugt im Bereich von 6.8 bis 7.2, gehalten und durch Kühlung von außen oder von innen eine Temperatur von 0° bis 10 °C, vorzugsweise von 5° bis 8 °C eingestellt. Die Zugabe von Natriumchlorid und Glycin nacheinander kann in beliebiger Form geschehen, doch hat es sich als zweckmäßig erwiesen, beide Proteinfällungsmittel in fester Form zuzugeben, um das Volumen der Lösung möglichst gering zu halten. Nach längerem Stehen, das je nach Größe des Ansatzes zwischen einer und mehreren Stunden betragen kann, wird der entstandene Niederschlag auf übliche Weise, bevorzugt durch Zentrifugieren, abgetrennt, wiedergelöst und durch Tieffrieren und Lyophilisieren in haltbare Form gebracht.

Zweckmäßig löst man dazu den das AHF-Konzentrat enthaltenden Niederschlag in der etwa fünf- bis sechsfachen Menge eines physiologischen Puffers, beispielsweise eines 0.1 Gew.%igen Tri-Natriumcitratpuffers, bei neutralem pH-Wert, wobei gelindes Erwärmen auf 30° bis 40 °C, bevorzugt auf 35 bis 37 °C, zur Beschleunigung des Lösungsvorgangs vorteilhaft sein kann. Diese Lösung kann zweckmäßig zur Entfernung allenfalls vorhandener niedermolekularer Anteile für mehrere Stunden gegen einen Puffer gleicher Konzentration und Ionenstärke wie der Lösungspuffer dialysiert werden. Die das AHF-Konzentrat enthaltende Lösung oder das Dialysat werden gegebenenfalls mit den für Faktor VIII üblichen Stabilisierungsmitteln, einzeln oder in Kombination, beispielsweise mit Natriumchlorid, Glycin, Tri-Natriumcitrat, Kohlenhydraten wie Glucose, oder Heparin versetzt, sterilfiltriert, abgefüllt und lyophilisiert.

Das erfindungsgemäße Verfahren ist spezifisch geeignet zur großtechnischen Herstellung eines gereinigten Faktor VIII-Konzentrats, welches allen Anforderungen in der modernen Therapie entspricht.

Das mit dem erfindungsgemäßen Verfahren hergestellte, lyophilisierte AHF-Konzentrat hat eine spezifische Aktivität von mindestens 1,5 E pro mg Protein und löst sich in Wasser rückstandsfrei zu einer injizierbaren Lösung mit mindestens 25 E AHF/ml Lösungsmittel.

Ein weiteres wesentliches Merkmal der Erfindung ist die überraschend hohe Ausbeute, mit der das über zwei Stufen gereinigte AHF-Konzentrat erhalten wird und die etwa 20 bis 30 % der theoretischen Plasmamenge beträgt.

Das folgende Beispiel dient der weiteren Erläuterung der Erfindung.

Beispiel :

6 kg Kryopräzipitat aus 596 l Plasma werden bei 35 °C in 30 l 0.1 Gew.%iger Tri-Natriumcitratpufferlösung gelöst. Nach dem Abkühlen der Lösung auf 21 °C wird mit 0.1 N HCl ein pH-Wert von 6.30 eingestellt. Anschließend wird auf 15 °C gekühlt, 30 min sedimentiert, der entstandene Niederschlag abzentrifugiert und der Überstand mit 0.1 N NaOH auf pH 6.95 eingestellt.

Zum Überstand werden bei 20 °C langsam portionsweise 5.5 kg Kochsalz zugegeben. Anschließend werden langsam portionsweise 3.5 kg Glycin zugegeben und während der Zugabe wird der pH-Wert kontrolliert und durch Zusatz von 0.1 N Natronlauge im Bereich von 6.9 bis 7.2 gehalten.

Die Mischung wird auf 8 °C abgekühlt und 2 Stunden lang gerührt. Anschließend wird der Niederschlag (607 g) durch Zentrifugieren abgetrennt und in ca. 3.2 l 0.1 Gew.%igem Tri-Natriumcitratpuffer gelöst. Diese Lösung wird 3 Stunden lang gegen 80 l 0.1 Gew.%igen Tri-Natriumcitratpuffer bei pH 7 dialysiert. Nach der Dialyse erhält man 4.2 l AHF-Lösung, die klärfiltriert wird. Anschließend wird Natriumchlorid, Glycin und Tri-Natriumcitrat zugegeben und in Flaschen zu je 20 ml abgefüllt. Die einzelnen Gefäße werden durch Lyophilisation vom Lösungsmittel befreit.

Man erhält ca. 266 Flaschen, die je Flasche 500 E. lyophilisiertes AHF-Konzentrat mit einer spez. Aktivität von mindestens 1,5 E FVIII/mg Protein enthalten, welches sich in Wasser zu einer klaren AHF-Lösung von mindestens 25 E AHF/ml rückstandsfrei löst.

## Patentansprüche

1. Verfahren zur Herstellung eines Antihämophiliefaktor-Konzentrats (AHF-Konzentrats) mit einer Konzentration von mindestens 25 E AHF/ml und einer spezifischen Aktivität von mindestens 1,5 E AHF/mg Protein aus menschlichem Blutplasma durch eine Kombination von Maßnahmen wie durch Kryopräzipitieren, Fällen, Reinigen mit Proteinfällungsmitteln, Zentrifugieren und Lyophilisieren, dadurch gekennzeichnet, daß man

a) das Kryopräzipitat in einem Puffer geringer Ionenstärke auflöst, in der Lösung einen pH-Wert von 6.0 bis 6.8 sowie eine Temperatur von 5 °C bis 15 °C einstellt und die dabei entstandene Fällung vom Überstand abtrennt, sodann

b) den so vorgereinigten und an AHF in Bezug auf die Begleitproteine angereicherten Überstand neutralisiert, durch aufeinanderfolgende Zugabe von Natriumchlorid bis zu einer Konzentration von etwa 15 Gew.% und von Glycin bis zu einer Menge von etwa 10 Gew.% einer Reinigungsfällung unterzieht, worauf der das AHF-Konzentrat enthaltende Niederschlag abgetrennt, gegebenenfalls unter Zusatz von Stabilisatoren in einem physiologischen Puffer wiedergelöst und durch Tieffrieren und Lyophilisieren in haltbare Form gebracht wird.

2. Verfahren nach Anspruch 1, dadurch gekennzeichnet, daß man die Fällung in Stufe a) bei einem pH-Wert von 6.30 und einer Temperatur von 10 °C bis 15 °C durchführt.

3. Verfahren nach den Ansprüchen 1 und 2, dadurch gekennzeichnet, daß man während der Glycinfällung in Stufe b) einen pH-Wert von 6.8 bis 7.2 einhält und eine Temperatur von 5 °C bis 8 °C einstellt.

4. Verfahren nach den Ansprüchen 1 bis 3, dadurch gekennzeichnet, daß man die physiologische Pufferlösung des AHF-Konzentrats in Stufe b) vor dem Lyophilisieren gegen eine Puffer gleicher Konzentration und Ionenstärke wie der Lösungspuffer dialysiert.

## Claims

1. Process for preparing an antihaemophilic factor concentrate (AHF concentrate) with a concentration of at least 25 U of AHF/ml and a specific activity of at least 1.5 U of AHF/mg of protein from human blood plasma by a combination of measures, such as by cryoprecipitation, precipitation, purification with protein precipitants, centrifugation and lyophilisation, characterized in that

a) the cryoprecipitate is dissolved in a buffer of low ionic strength, the solution is adjusted to a pH of 6.0 to 6.8 and to a temperature of 5 °C to 15 °C, and the precipitate resulting thereby is separated from the supernatant, and then

b) the supernatant, which has been pre-purified in this way and enriched in AHF in relation to the concomitant proteins, is neutralised, and is subjected to a purifying precipitation by successive addition of sodium chloride up to concentration of about 15 % by weight and of glycine up to an amount of about 10 % by weight, whereupon the precipitate containing the AHF concentrate is separated off, redissolved in a physiological buffer, where appropriate with the addition of stabilisers, and converted by deep-freezing and lyophilisation into a form which can be stored.

2. Process according to Claim 1, characterized in that the precipitation in stage a) is carried out at a pH of 6.30 and a temperature of 10 °C to 15 °C.

3. Process according to Claims 1 and 2, characterized in that a pH of 6.8 to 7.2 is maintained, and the temperature is adjusted to 5 °C to 8 °C, during the glycine precipitation in stage b).

4. Process according to Claims 1 to 3, characterized in that the physiological buffer solution of AHF concentrate in stage b) is, before lyophilisation, dialysed against a buffer of the same concentration and ionic strength as the dissolving buffer.

## Revendications

1. Procédé de préparation d'un concentré de facteur antihémophilique (concentré d'AHF) d'une concentration d'au moins 25 U AHF/ml et d'une activité spécifique d'au moins 1,5 U AHF/mg de protéine, à partir de plasma du sang humain, par une combinaison de mesures telles que la cryoprécipitation, la précipitation, la purification par des précipitants de protéines, la centrifugation et la lyophilisation, caractérisé en ce que :

a) on dissout le cryoprécipité dans un tampon de faible force ionique, on ajuste dans la solution une valeur de pH de 6,0 à 6,8, ainsi qu'une température de 5 °C à 15 °C et l'on sépare le précipité alors formé du surnageant, puis

b) on neutralise le surnageant ainsi prépurifié et enrichi en AHF par rapport aux protéines d'accompagnement, on lui fait subir par addition successive de chlorure de sodium jusqu'à une concentration d'environ 15 % en poids et de glycine jusqu'à une proportion d'environ 10 % en poids une précipitation de purification, en séparant alors le précipité renfermant le concentré d'AHF, on le redissout éventuellement en ajoutant des stabilisants dans un tampon physiologique et on le met sous forme conservable par surgélation et lyophilisation.

2. Procédé suivant la revendication 1, caractérisé en ce qu'on effectue la précipitation du stade a) à une valeur de pH de 6,30 et une température de 10 °C à 15 °C.

3. Procédé suivant les revendications 1 et 2, caractérisé en ce qu'on maintient, pendant la précipitation par la glycine du stade b) une valeur de pH de 6,8 à 7,2, et l'on établit une température de 5 °C à 8 °C.

4. Procédé suivant les revendications 1 à 3, caractérisé en ce qu'on dialyse la solution-tampon physiologique du concentré d'AHF du stade b) avant la lyophilisation contre un tampon de même concentration et de même force ionique que le tampon de dissolution.